# EUROPÄISCHE PATENTSCHRIFT

(11) **EP 1 033 963 B1**
(45) Veröffentlichungstag und Bekanntmachung des Hinweises auf die Patenterteilung: **13.09.2006**
(21) Anmeldenummer: 98956916.5
(22) Anmeldetag: 16.11.1998
(51) Int. Cl.: A61K 8/29, A61Q 5/00, A61Q 17/04

(54) **TRÜBUNGSMITTEL**
OPACIFIER
OPACIFIANT

(30) Priorität: 24.11.1997 DE 19751954
(43) Veröffentlichungstag der Anmeldung: 13.09.2000
(73) Patentinhaber: Henkel Kommanditgesellschaft auf Aktien, 40589 Düsseldorf (DE)
(72) Erfinder: PRIEBE, Christian, D-42489 Wülfrath (DE); GODDINGER, Dieter, D-25336 Klein Nordende (DE)
(86) Internationale Anmeldenummer: PCT/EP1998/007302
(87) Internationale Veröffentlichungsnummer: WO 1999/026593

(56) Entgegenhaltungen:
- US-A- 3 956 163
- US-A- 5 441 728

## Beschreibung

Die Erfindung betrifft ein Verfahren zur gezielten Eintrübung flüssiger, gelförmiger oder emulsionsförmiger, wäßriger Zubereitungen durch Zusatz von feinteiligem Polyvinylacetat oder eines Copolymers aus Vinylacetat und anderen Vinylestern.

Wäßrige Zubereitungen zur Reinigung und Pflege der Haut oder der Haare liegen oftmals in Form von klaren oder transparenten flüssigen oder gelförmigen Zubereitungen vor. Auch emulsionsförmige Zubereitungen weisen nicht immer eine dichte und gehaltvolle Trübung auf, insbesondere die stabilen, feinteiligen Emulsionen und Mikroemulsionen sind meist durchscheinend bis opaleszent und wirken dann wenig gehaltvoll.

Viele Verwender bevorzugen aber solche kosmetischen Zubereitungen, die ein intensiv milchig-weißes Aussehen haben, wie es üblicherweise nur bei grobteiligen, konzentrierten Emulsionen auftritt, da ein solches Aussehen der erwarteten Pflegewirkung besser entspricht.

Es ist daher seit langem üblich, z.B. Pflegelotionen durch emulgierte Fettstoffe ein weißes, milchiges Aussehen zu verleihen. Solche emulgierten oder dispergierten Fettstoffe, z.B. langkettige Fettalkohole, Fettsäuren, Fettsäureglycolester, Fettsäuremonoglyceride, Fett-säurealkanolamide oder Magnesiumstearat erzeugen z.T. sogar einen Perlglanzeffekt, der bei einigen Zubereitungen auch erwünscht ist.

In vielen Fällen ist aber ein solcher Perlglanz oder ein hoher Anteil an emulgierten oder dispergierten Fettkomponenten aus ästhetischen oder anwendungstechnischen Gründen unerwünscht. Auch können grobteilige Emulsionen oder Dispersionen von Fettkomponenten leicht zu Stabilitätsproblemen führen, die ein Absetzen oder Aufrahmen der Trübung zur Folge haben.

Man hat daher seit langem auch schon Polymerlatizes, z. B. Suspensionen von Polyacrylaten oder von Polystyrolen als Trübungsmittel eingesetzt. Leider erzeugen solche Trübungsmittel oftmals einen wenig befriedigenden und künstlich wirkenden Eindruck. Ein weiterer Nachteil der bekannten Styrol-/Acrylat-Trübungsmittel ist ihre relativ schlechte biologische Abbaubarkeit. Es bestand daher die Aufgabe, ein Trübungsmittel zu finden, das in relativ geringer Einsatzkonzentration eine stabile, dichte und gehaltvoll aussehende Trübung in flüssigen, gelförmigen und emulsionsförmigen Zubereitungen erzeugt und das außerdem problemlos in seinem ökologischen Verhalten, insbesondere bezüglich der biologischen Abbaufähigkeit, ist.

Es wurde nunmehr gefunden, daß sich Polyvinylacetat und Copolymere aus Vinylacetat und anderen Vinylestern zur Lösung der gestellten Aufgabe eignen.

Gegenstand der Erfindung ist daher ein Verfahren zur Eintrübung flüssiger, gelförmiger oder emulsionsförmiger wäßriger Zubereitungen durch Zugabe feinteiliger Polymerisate, wobei man als Polymerisate Polyvinylacetat oder Copolymere aus Vinylacetat und anderen Comonomeren (PVAc-Copolymere) zusetzt.

Die erfindungsgemäß geeigneten Polymerisate können als wäßrige Dispersionen (Latices) oder in Form von feinteiligen, wasserdispergierbaren Pulvern eingesetzt werden. Die Latices weisen meist einen Feststoffgehalt von 40 - 60 Gew.-% Polyvinylacetat auf. Obwohl Dispersionen oder PVAc-Pulver mit einer relativ hohen Teilchengröße eine intensive Trübung bewirken, sind diese nicht immer erwünscht, da sie in kosmetischen Zubereitungen bei üblichen Viskositäten schwer zu stabilisieren sind (Absetzen/Aufrahmen). Im allgemeinen werden daher Polymerisate mit einer Teilchengröße von 100 bis 5000 nm (Nanometer) bevorzugt. Bevorzugt sollen wenigstens ca. 90 Gew.-% des Polymerisats aus Teilchen mit einem Durchmesser von 100 - 5000 nm bestehen.

Obwohl Vinylacetat-Homopolymerisate bevorzugt sind, eignen sich auch Copolymerisate mit anderen Comonomeren. Als Comonomere können z.B. Ethylen, Acrylsäureester oder Styrol, bevorzugt aber Vinylester von C₃-C₃₀-Monocarbonsäuren enthalten sein. Ein geeignetes Comonomer ist z.B. Vinyllaurat. Der Gehalt an Comonomeren im Copolymerisat sollte jedoch nicht mehr als 20 Gew.-% des Copolymers ausmachen.

Die erfindungsgemäß geeigneten feinteiligen Polymerisate sind bevorzugt frei von äußeren Weichmachern und enthalten in der Regel ein Schutzkolloid, meistens Polyvinylalkohol, zur Stabilisierung. Solche Produkte wurden bisher z.B. zur Herstellung von Leimen und Kleistern, als Zusatz zu Spachtelmassen und Kitten, als Zusatz zu Apparaturen und zu hydraulischen Bindemitteln verwendet.

Vinylacetat-Homopolymerisate mit der bevorzugten Teilchengröße sind handelsüblich und werden von verschiedenen Herstellern angeboten, z.B. von Hoechst AG unter dem Warenzeichen Mowilith® D, DC oder DN, von Wacker-Chemie GmbH unter dem Warenzeichen Vinnapas® D50R oder von Cordes & Co. GmbH unter dem Warenzeichen Wormalit® PM.

Die Menge des für die Trübung einzusetzenden Polyvinylacetats oder PVAc-Copolymers richtet sich natürlich in erster Linie nach der gewünschten Intensität der Trübung. Es wurde aber festgestellt, daß Polyvinylacetat-Dispersionen oder redispergierbare PVAc-Pulver sehr ergiebige Trübungsmittel sind und man schon durch einen Zusatz von 0,5 Gew.-% (Feststoff) an Polyvinylacetat oder PVAc-Copolymer eine deutliche Eintrübung erhält, mit Zusätzen von 1 - 5 Gew.-% (Feststoff) des Trübungsmittels werden im allgemeinen sehr befriedigende Trübungen erhalten.

Bevorzugt ist also das erfindungsgemäße Verfahren, bei dem man Polyvinylacetat oder ein Copolymeres aus Vinylacetat und einem Vinylester einer C₃-C₃₀-Monocarbonsäure in Form einer wäßrigen Dispersion oder eines wasserdispergierbaren Pulvers mit einer Teilchengröße von 100 - 5000 nm in einer Menge von wenigstens 0,5 Gew.-% der gesamten Zubereitung zusetzt.

Gegenstand der Erfindung ist weiterhin die Verwendung von Polyvinylacetat oder Copolymeren aus Vinylacetat und Vinylestern von C₃-C₃₀-Carbonsäuren in Form wäßriger Dispersionen oder wasserdispergierbarer Pulver zur Eintrübung flüssiger, gelförmiger oder emulsionsförmiger Zubereitungen.

Die einzutrübenden Zubereitungen dienen bevorzugt der Reinigung und Pflege des Körpers oder der Haare, d.h. es handelt sich z.B. um Flüssigseifen, Shampoos, Schaumbäder, Duschgele, Haarspülmittel, Haut- und Gesichtsreinigungslotionen, Hautpflegelotionen und andere, bevorzugt im kosmetischen Bereich verwendete Zubereitungen. Die erfindungsgemäße Verwendung erstreckt sich aber auch auf die Eintrübung anderer, vornehmlich im Haushalt eingesetzter Zubereitungen wie z.B. Geschirrspülmittel, Fußboden- und Allzweckreiniger, flüssige Waschmittel, Sanitärreiniger und andere flüssige, gelförmige oder emulsionsförmige Zubereitungen.

Die einzutrübenden Zubereitungen zeichnen sich in der Regel durch einen Gehalt an oberflächenaktiven Stoffen aus. Diese können anionisch, kationisch, zwitterionisch, ampholytisch oder nichtionisch sein, ohne daß es zu Wechselwirkungen oder Instabilitäten der durch Polyvinylacetat erzeugten Trübungen kommt. Im allgemeinen liegt der Gehalt an Tensiden im Bereich von 1 - 20 Gew.-%. Zur Herstellung von Gelen können darüber hinaus wasserlösliche Hydrokolloide, z.B. Pflanzengumme, lösliche Stärke, Celluloseether, Biopolymere (z.B. Xanthan-Gum) oder synthetische wasserlösliche Polymere, bevorzugt in einer Menge von 0,5 - 5 Gew.-%, enthalten sein. Auch Emulsionen vom Öl-in-Wasser-Typ oder sogar Wasser-in-Öl-Emulsionen, die keine ausreichende Trübung aufweisen, können nach der Erfindung durch Zusatz von Polyvinylacetat in feinteiliger Form eingetrübt werden. Im Falle der Öl-in-Wasser-Emulsion genügt es, eine Polyvinylacetat-Dispersion der Emulsion zuzugeben, die sich dann in der äußeren wäßrigen Phase verteilt. Im Falle der Wasser-in-Öl-Emulsion sollte man entweder zuerst die Wasserphase mit der Dispersion eintrüben oder der fertigen W/O-Emulsion ein Polyvinylacetat-Pulver zusetzen, das sich dann in der äußeren Phase dispergiert.

Ein bevorzugter Erfindungsgegenstand sind wäßrige, flüssige oder emulsionsförmige Zubereitungen zur Reinigung und Pflege der Haut oder Haare mit einem Gehalt an oberflächenaktiven Stoffen, Hydrocolloiden oder Gemischen davon, die als Trübungsmittel ein dispergiertes, feinteiliges Polyvinylacetat in einer Menge von 0,5 - 5 Gew.-%, bezogen auf die gesamte Zubereitung, enthalten.

Die folgenden Beispiele sollen den Erfindungsgegenstand näher erläutern:

### Beispiele

Es wurden die folgenden Handelsprodukte verwendet:

### 1. Trübungsmittel

| | |
|---|---|
| Wormalit® PM 5208 : | (Cordes & Co. GmbH, D-32457 Porta Westfalica, DE) |
| Polyvinylacetat-Dispersion | |
| Feststoffgehalt | : 55 Gew.-% |
| mittlere Teilchengröße : | ca. 2000 nm |

| | |
|---|---|
| Wormalit® PM 4239 : | (Cordes & Co. GmbH, D-32457 Porta Westfalica, DE) |
| Polyvinylacetat-Dispersion | |
| Feststoffgehalt : | 55Gew.-% |
| mittlere Teilchengröße | : ca. 1000 nm |

| | |
|---|---|
| Vinnapas® D 50 R : | (Wacker-Chemie GmbH, D-81737 München) |
| Polyvinylacetat, redispergierbares Dispersionspulver | |
| Feststoffgehalt : | 99 ± 1 Gew.-% |
| Teilchengröße : | 1000 - 5000 µm |

| | |
|---|---|
| Mowilith® DN : | (Hoechst AG, Frankfurt am Main, DE) |
| Polyvinylacetat-Dispersion | |
| Feststoffgehalt : | 50 ± 1 Gew.-% |
| Teilchengröße (90 %) : | 100 - 1500 nm |

| | |
|---|---|
| Mowilith® DC : | (Hoechst AG, Frankfurt / Main, DE) |
| Polyvinylacetat-Dispersion | |
| Feststoffgehalt : | 56 ± 1 Gew.-% |
| Teilchengröße (90 %) : | 300 - 2000 nm |

### 2. Tenside

| | |
|---|---|
| Texapon® NSO : | (Henkel KGaA, Düsseldorf, DE) |
| Natriumlaurylethersulfat | |
| Aktivsubstanzgehalt : | 28 Gew.-% |

| | |
|---|---|
| Dehyton® K : | (Henkel KGaA, Düsseldorf, DE) |
| Kokosfettacylamidopropyl-Betain | |
| Aktivsubstanzgehalt : | 30 Gew.-% |

| | |
|---|---|
| Plantacare® 818 : | (Henkel KGaA, Düsseldorf, DE) |
| C₈-C₁₄-Alkylpoly-(1,4)-glucosid | |
| Aktivsubstanzgehalt : | ca. 52 Gew.-% |

| | |
|---|---|
| Rewoteric® AM 2 CNM : | (WITCO-Surfactants GmbH, (Rewo)) |
| Kokosfettacylamidoethyl-N-2-hydroxyethylglycinat, Na-Salz | |
| Aktivsubstanzgehalt : | ca. 40 Gew.-% |

| | |
|---|---|
| Eumulgin® B2 : | (Henkel KGaA, Düsseldorf, DE) |
| Cetyl-/Stearylalkohol-poly-(20)-glycolether | |

### 3. Fettstoffe

| | |
|---|---|
| Cutina® GMS : | (Henkel KGaA, Düsseldorf, DE) |
| Glycerin-mono/di-palmitat-/stearat | |
| Monoglyceridgehalt : | ca. 50 Gew.-% |

| | |
|---|---|
| Steonol® 1618 : | (Henkel KGaA, Düsseldorf, DE) |
| Cetyl-/Stearylalkohol | |

| | |
|---|---|
| Cetiol® OE : | (Henkel KGaA, Düsseldorf, DE) |
| Di-n-Octylether | |

| | |
|---|---|
| Cutina® CP : | (Henkel KGaA, Düsseldorf, DE) |
| Cetylpalmitat | |

| | |
|---|---|
| Lameform® TGI : | (Henkel KGaA, Düsseldorf, DE) |
| Triglycerin-diisostearat | |

### 4. Hydrocolloide

| | |
|---|---|
| Carbopol® ETD 2001 : | (BF Goodrich) |
| Polyacrylsäure (Homopolymer) | |

| | |
|---|---|
| Keltrol® RD : | (Kelco) |
| Xanthan-Gum | |

### Ausführungsbeispiele

### Tensidzubereitungen

### 1.

| | |
|---|---|
| Texapon® NSO | 40,0 Gew.-% |
| Dehyton® K | 10,0 Gew.-% |
| Plantacare® 818 | 2,0 Gew.-% |
| Wormalit® PM 5208 | 2,0 Gew.-% |
| Natriumchlorid | 1,5 Gew.-% |
| Wasser | 44,5 Gew.-% |

Es wird eine samtweiße Lotion erhalten. Ohne den Zusatz der Polyvinylacetat-Dispersion ist die Zubereitung klar und farblos.

### 2.

| | |
|---|---|
| Texapon® NSO | 40,0 Gew.-% |
| Rewoteric® AC 2 CNM | 7,0 Gew.-% |
| Vinnapas® D 50 R | 2,5 Gew.-% |
| Wasser | 50,5 Gew.-% |

### 3. Öl-in-Wasser-Emulsion

| | |
|---|---|
| Stenol® 1618 | 4,8 Gew.-% |
| Cutina® GMS | 0,5 Gew.-% |
| Eumulgin® B2 | 0,4 Gew.-% |
| Mowilith® DN | 3,0 Gew.-% |
| Wasser | ad 100 Gew.-% |

### 4. Wasser-in-Öl-Emulsion

| | |
|---|---|
| Stenol® 1618 | 4,7 Gew.-% |
| Cetiol® OE | 1,0 Gew.-% |
| Cutina® CP | 2,0 Gew.-% |
| Lameform® TGI | 3,0 Gew.-% |
| Wormalit® PM 4239 | 3,0 Gew.-% |
| Wasser | ad 100 Gew.-% |

### 5. Gelformulierung

| | |
|---|---|
| Carbopol® ETD 2001 | 0,8 Gew.-% |
| Natronlauge (10 %ig) | bis pH = 7,0 |
| Mowilith® DC | 2,5 Gew.-% |
| Wasser | ad 100 Gew.-% |

### 6. Gelformulierung

| | |
|---|---|
| Keltrol® RD | 1,0 Gew.-% |
| Wormalit® PM 5208 | 1,4 Gew.-% |
| Wasser | 97,6 Gew.-% |

## Patentansprüche

1. Verfahren zur Eintrübung flüssiger, gelförmiger oder emulsionsförmiger wäßriger Zubereitungen durch Zugabe feinteiliger Polymerisate mit einer Teilchengröße von 100 - 5000 nm, **dadurch gekennzeichnet, daß** man als Polymerisate Polyvinylacetat oder Copolymere aus Vinylacetat und anderen Comonomeren, wobei der Gehalt an Comonomeren im Copolymerisat nicht mehr als 20 Gew.-% ausmacht, zusetzt.

2. Verfahren nach Anspruch 1, **dadurch gekennzeichnet, daß** man Polyvinylacetat oder ein Copolymer aus Vinylacetat und einem Vinylester einer C₃-C₃₀-Carbonsäure in Form einer wäßrigen Dispersion oder eines wasserdispergierbaren Pulvers mit einer Teilchengröße von 100 - 5000 nm in einer Menge von wenigstens 0,5 Gew.-% der gesamten Zubereitung zusetzt.

3. Verwendung von Polyvinylacetat oder Copolymeren aus Vinylacetat und anderen Comonomeren, wobei der Gehalt an Comonomeren im Copolymerisat nicht mehr als 20 Gew.-% ausmacht, mit einer Teilchengröße von 100 - 5000 nm zur Eintrübung flüssiger, gelförmiger oder emulsionsförmiger wäßriger Zubereitungen.

4. Verwendung nach Anspruch 3, **dadurch gekennzeichnet, daß** die Polymerisate in Form wäßriger Dispersionen oder feinteiliger wasser-dispergierbarer Pulver eingesetzt werden.

5. Verwendung nach einem der Ansprüche 3 oder 4, **dadurch gekennzeichnet, daß** als Polymerisate Polyvinylacetat oder Copolymere aus Vinylacetat und Vinylestern von C₃₋C₃₀-Monocarbonsäuren eingesetzt werden.

6. Wäßrige, flüssige, gelförmige oder emulsionsförmige Zubereitungen zur Reinigung und Pflege der Haut oder der Haare mit einem Gehalt an Hydrocolloiden und gegebenenfalls oberflächenaktiven Stoffen, **dadurch gekennzeichnet, daß** sie als Trübungsmittel ein dispergiertes, feinteiliges Polyvinylacetat oder Copolymere aus Vinylacetat und anderen Comonomeren, wobei der Gehalt an Comonomeren im Copolymerisat nicht mehr als 20 Gew.-% ausmacht, mit einer Teilchengröße von 100 - 5000 nm in einer Menge von 0,5 bis 5 Gew.-%, bezogen auf die gesamte Zubereitung, enthalten.

7. Zubereitungen nach Anspruch 6, **dadurch gekennzeichnet, daß** sie als Trübungsmittel ein dispergiertes, feinteiliges Polyvinylacetat oder Copolymere aus Vinylacetat und Vinylestern von C₃-C₃₀-Monocarbonsäuren enthalten.

8. Zubereitungen nach einem der Ansprüche 6 oder 7, **dadurch gekennzeichnet, daß** sie als Trübungsmittel ein dispergiertes, feinteiliges Polyvinylacetat enthalten.

## Claims

1. A process for opacifying liquid, gel-form or emulsion-form water-containing preparations by addition of fine-particle polymers with a particle size of 100 to 5,000 nm, **characterized in that** polyvinyl acetate or copolymers of vinyl acetate and other comonomers, the comonomer content of the copolymer being no more than 20% by weight, are added as polymers.

2. A process as claimed in claim 1, **characterized in that** polyvinyl acetate or a copolymer of vinyl acetate and a vinyl ester of a C₃₋₃₀ carboxylic acid in the form of an aqueous dispersion or a water-dispersible powder with a particle size of 100 to 5,000 nm is added in a quantity of at least 0.5% by weight of the preparation as a whole.

3. The use of polyvinyl acetate or copolymers of vinyl acetate and other comonomers, the comonomer content of the copolymer being no more than 20% by weight, with a particle size of 100 to 5,000 nm for opacifying liquid, gel-form or emulsion-form water-containing preparations.

4. The use claimed in claim 3, **characterized in that** the polymers are used in the form of aqueous dispersions or fine-particle water-dispersible powders.

5. The use claimed in claim 3 or 4, **characterized in that** polyvinyl acetate or copolymers of vinyl acetate and vinyl esters of C₃₋₃₀ monocarboxylic acids are used as the polymers.

6. Water-containing, liquid, gel-form or emulsion-form skin- or hair-care and -cleaning preparations containing hydrocolloids and optionally surfactants, **characterized in that** they contain as opacifier a dispersed, fine-particle polyvinyl acetate or copolymers of vinyl acetate and other comonomers, the comonomer content of the copolymer being no more than 20% by weight, with a particle size of 100 to 5,000 nm in a quantity of 0.5 to 5% by weight, based on the preparation as a whole.

7. Preparations as claimed in claim 6, **characterized in that** they contain a dispersed, fine-particle polyvinyl acetate or copolymers of vinyl acetate and vinyl esters of C₃₋₃₀ monocarboxylic acids as opacifier.

8. Preparations as claimed in claim 6 or 7, **characterized in that** they contain a dispersed fine-particle polyvinyl acetate as opacifier.

## Revendications

1. Procédé pour opacifier des préparations aqueuses liquides, sous forme de gel ou d'émulsion, par addition de polymères finement divisés ayant une taille particulaire allant de 100 à 5000 nm, **caractérisé en ce qu'**on ajoute comme polymères l'acétate de polyvinyle ou des copolymères d'acétate de vinyle et d'autres comonomères, la teneur en comonomères dans le copolymère ne dépassant pas 20 % en poids.

2. Procédé selon la revendication 1, **caractérisé en ce qu'**on ajoute de l'acétate de polyvinyle ou un copolymère d'acétate de vinyle et d'un ester vinylique d'un acide carboxylique en C₃ à C₃₀ sous la forme d'une dispersion aqueuse ou d'une poudre dispersible dans l'eau avec une taille particulaire de 100 à 5000 nm en une quantité d'au moins 0,5 % en poids de l'ensemble de la préparation.

3. Utilisation d'acétate de polyvinyle ou de copolymères d'acétate de vinyle et d'autres comonomères, où la teneur en comonomères dans le copolymère ne dépasse pas 20 % en poids, avec une taille particulaire allant de 100 à 5000 nm pourt opacifier des préparations aqueuses liquides, sous forme de gel ou d'émulsion.

4. Utilisation selon la revendication 3, **caractérisée en ce que** les polymères sont utilisés sous forme de dispersions aqueuses ou de poudres finement divisées dispersibles dans l'eau.

5. Utilisation selon l'une des revendications 3 ou 4, **caractérisée en ce qu'**on utilise comme polymères l'acétate de polyvinyle ou des copolymères d'acétate de vinyle et d'esters vinyliques d'acides monocarboxyliques en C₃ à C₃₀.

6. Préparations aqueuses liquides, sous forme de gel ou d'émulsion pour le nettoyage et le soin de la peau ou des cheveux, contenant des hydrocolloïdes et le cas échéant des substances tensioactives, **caractérisées en ce qu'**elles contiennent comme agent opacifiant un acétate de polyvinyle finement divisé, dispersé, ou des copolymères d'acétate de vinyle et d'autres comonomères, la teneur en comonomères dans le copolymère ne dépassant pas 20 % en poids, avec une taille particulaire de 100 à 5000 nm en une quantité de 0,5 à 5 % en poids, par rapport à l'ensemble de la préparation.

7. Préparations selon la revendication 6, **caractérisées en ce qu'**elles contiennent comme agent opacifiant un acétate de polyvinyle finement divisé, dispersé, ou des copolymères d'acétate de vinyle et d'esters vinyliques d'acides monocarboxyliques en C₃ à C₃₀.

8. Préparations selon l'une des revendications 6 ou 7, **caractérisées en ce qu'**elles contiennent comme agent opacifiant un acétate de polyvinyle finement divisé, dispersé.
